(19) Europäisches Patentamt
European Patent Office
Office européen des brevets

(11) Publication number: **0 433 911 A1**

(12) # EUROPEAN PATENT APPLICATION

(21) Application number: **90124243.8**

(51) Int. Cl.$^5$: **A61K 7/32**

(22) Date of filing: **14.12.90**

(30) Priority: **22.12.89 JP 334289/89**

(43) Date of publication of application: **26.06.91 Bulletin 91/26**

(84) Designated Contracting States: **AT CH DE LI NL**

(71) Applicant: **KAO CORPORATION**
**14-10, Nihonbashi Kayabacho 1-chome**
**Chuo-ku Tokyo(JP)**

(72) Inventor: **Ueda, Masayuki**
**Kao Dormitory, 1-3, Asahigaoka**
**Chiba-shi, Chiba-ken(JP)**
Inventor: **Tokimitsu, Ichiro**
**7-23-616, Shin Ogawa-machi**
**Shinjuku-ku, Tokyo(JP)**
Inventor: **Arisawa, Masatoshi**
**523-8, Koyama**
**Matsudo-shi, Chiba-ken(JP)**

(74) Representative: **Patentanwälte Grünecker, Kinkeldey, Stockmair & Partner**
**Maximilianstrasse 58**
**W-8000 München 22(DE)**

(54) **Deodorant for axillary odor.**

(57) A deodorant for axillary odor comprising glycyrrhetic acid.

EP 0 433 911 A1

# DEODORANT FOR AXILLARY ODOR

## BACKGROUND OF THE INVENTION

[Field of the Invention]

This invention relates to a deodorant for axillary odor which comprises glycyrrhetic acid and is particularly effective for preventing pungent axillary odor.

[Description of the Prior Art]

Although the causes for axillary odor have never been clarified yet in detail, it is considered that axillary odor would be caused by some functions of microorganisms on secretions discharged from sebaceous, apocrine or eccrine glands [refer to, for example J. Invest. Derm., 77, 413 - 416 (1981)].

There have been a number of products aiming at preventing or relieving axillary odor. Many of these products usually comprise, for example, antiperspirants, bactericides, agents for masking odoriferous components and adsorbents.

As the above-mentioned antiperspirants for controlling perspiration, astringents comprising aluminum compounds are employed in most cases and aluminum chloride may be cited as a common example thereof.

As the above-mentioned bactericides which control the growth of microorganisms causing axillary odor, hexachlorophene and various quaternary ammonium compoundsare used.

As the above-mentioned agents for masking odoriferous components, those having a pleasant smell such as eugenol are used.

Furthermore, examples of the above-mentioned adsorbents for odoriferous components include zinc oxide, activated carbon and zeolite, as disclosed in Japanese Patent Laid-open No. 43665/1988.

Although the above-mentioned antiperspirants can decrease the amount of sweat, namely, the source of sweat-door components, it is impossible, from a physiological viewpoint, to completely suppress perspiration. Further, these antiperspirants are hardly effective on pungent axillary odor, namely, so-called apocrine odor.

In addition, it has been pointed out that the above-mentioned bactericides suffer from problems in safety, which makes it impossible to use these substances at such a high concentration as to fully exert their effects.

Further, it is sometimes observed that the above-mentioned masking agents give an unpleasant smell when mixed with axillary odor.

Although the above-mentioned adsorbents are effective on low boiling point components such as lower fatty acids, they are not so effective on high boiling point ones which cause axillary odor partly.

As described above, each of the ingredients employed in conventional products aiming at preventing (or relieving) axillary odor has some disadvantages. Therefore, conventional products comprising these ingredients cannot give a satisfactory effect of preventing axillary odor.

## SUMMARY OF THE INVENTION

It is an object of the present invention to provide an axillary odor-preventing agent which is highly effective for preventing pungent axillary odor, namely, so-called apocrine odor, and suffers from no problem in safety.

The present inventors have conducted extensive studies in order to achieve the above object. As a result, they have found out that glycyrrhetic acid is highly effective in preventing pungent axillary odor.

Accordingly the present invention, which has been completed based on the above finding, provides an apocrine odor-preventing agent comprising glycyrrhetic acid.

## DETAILED DESCRIPTION OF THE INVENTION

The glycyrrhetic acid to be used in the present invention, which is the main ingredient of licoric root widely used in the field of Chinese medicines, is represented by the following formula:

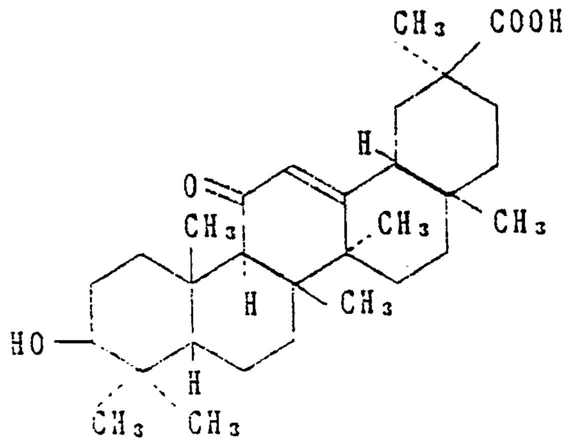

( I ).

When the above glycyrrhetic acid is to be used together with other ingredients, the content of the glycyrrhetic acid is not specifically restricted, so long as axillary odor can be effectively relieved thereby. The content of glycyrrhetic acid in the final preparation may preferably range from 0.01 to 5% by weight, still preferably from 0.1 to 3% by weight, though it may vary depending on the combination with other ingredients as well as the formulation of the final preparation. When the content of glycyrrhetic acid is smaller than 0.01% by weight, a sufficient effect can be hardly achieved. When it exceeds 5% by weight, on the other hand, some problems in the formulation, for example, crystallization, might be observed.

In addition to glycyrrhetic acid, the deodorant for axillary odor of the present invention may further comprise other ingredients for preventing body odor selected from among those commonly employed in the art. Preferable examples thereof include antiperspirants such as aluminum hydroxychloride, aluminum chloride, aluminum sulfate, basic aluminum bromide, aluminum phenolsulfonate, tannic acid, aluminum naphthalenesulfonate and basic aluminum iodide; bactericides such as 2,4,4'-trichloro-2'-hydroxydiphenyl ether (Irgasan DP-300), 3,4,4-trichlorocarbanilide (TCC), benzalkonium chloride, benzethonium chloride, alkyltrimethylammonium chlorides, resorcin, phenol, sorbic acid, salicylic acid and hexachlorophene; and masking agents such as musk, skatole, lemon oil, lavender oil, absolute, jasmine, vanillin, benzoin, benzyl acetate and menthol.

The deodorant for axillary odor of the present invention may further comprise, depending on the formulation, various additives such as oils, surfactants, water, alcohols, saccharides, esters, preservatives, vitamins such as vitamin A, vitamin D, vitamin E and vitamin K, hormones such as estradiol and cortisone, anti-inflammatory agents such as allantoin, humectants, thickeners, perfumes and colorants within such a range as not to deteriorate the effects of the present invention.

The deodorant for axillary odor of the present invention may be formulated into, for example, aerosol, roll-on, powder, cream, lotion, stick, solid detergent or liquid detergent. For example, it may be applied to the axillary parts one to three times per day followed by allowing to stand as such or washing away.

To further illustrate the present invention, and not by way of limitation, the following Examples will be given.

Example 1

Table 1: Composition of deodorant lotion (% by weight)

| Ingredient | Invention product 1 | Comparative product | |
|---|---|---|---|
| | | 1 | 2 |
| β-glycyrrhetic acid | 1.0 | - | - |
| Irgasan DP-300* | 0.2 | - | 0.2 |
| aluminum hydroxychloride | 10.0 | 10.0 | 10.0 |
| propylene glycol | 5.0 | 5.0 | 5.0 |
| ethanol | 10.0 | 10.0 | 10.0 |
| perfume | 0.1 | 0.1 | 0.1 |
| purified water | balance | balance | balance |
| in total | 100.0 | 100.0 | 100.0 |

*: 2,4,4'-trichloro-2'-hydroxydiphenyl ether.

A deodorant lotion of each composition as specified in the above Table 1 was produced in a conventional manner. Then these products were subjected to the following test. The obtained results are as follows.

[Production method]

Aluminum hydroxychloride and propylene glycol were dissolved in purified water to thereby give an aqueous phase. On the other hand, other ingredients were dissolved in ethyl alcohol to thereby give an alcoholic phase. The alcoholic phase was added to the aqueous phase. After filtering, a deodorant lotion was obtained.

[Test Method]

The effect of preventing axillary odor was examined by using subjects suffering from serious axillary odor. The axillary odor of each subject was evaluated according to the following criteria before and after the application of each deodorant lotion. The test period was two weeks and the application was effected twice a day.

(Evaluation criteria)

Serious axillary odor: + +.
Moderate axillary odor: +.
Slight axillary odor: ±.
No axillary odor; -.

[Results]

Table 2 summarizes the results.

In Table 2, "effective" means a case wherein the score after the application is lower by two grades than the one before the application; "somewhat effective" means a case wherein the former score is lower by one grade than the latter one; and "ineffective" means other cases.

As Table 2 shows, the invention product 1 shows a remarkable effect of improving axillary odor, compared with the comparative products 1 and 2.

Table 2 (No. of subjects)

| | | Effective | Somewhat effective | Ineffective |
|---|---|---|---|---|
| Invention product 1 | | 2 | 4 | 4 |
| Comparative product | 1 | 0 | 2 | 8 |
| | 2 | 0 | 3 | 7 |

Example 2

A deodorant powder of the composition as specified in Table 3 was produced in the following manner. The deodorant powder thus obtained (the invention product 2) was then subjected to the test as specified below. Thus the following results were obtained.

Table 3 (Composition of deodorant powder: % by weight)

| Ingredient | Invention product 2 |
|---|---|
| β-glycyrrhetic acid | 2.0 |
| zinc oxide | 3.0 |
| talc*1 | 92.9 |
| liquid paraffin*2 | 2.0 |
| perfume | 0.1 |
| in total | 100.0 |

*1: Trademark: #5141, Lowmicron Alpine talc.

*2: Trademark: Silicol P-80.

[Production Method]

Zinc oxide and talc were well mixed together in a blender and a perfume and glycyrrhetic acid dispersed in liquid paraffin were uniformly sprayed thereto. After grinding and compression molding, a deodorant powder was obtained.

[Test Method]

The effect of the invention product 2 of preventing axillary odor was examined by using subjects suffering from serious axillary odor. The invention product 2 was applied to one axillary part of each subject while a control deodorant powder, which comprised the same ingredients as those of the invention product 2 except glycyrrhetic acid, was applied to another axillary part. The axillary odor was evaluated in the same manner as the one described in Example 1. The test period was two weeks and the application was effected twice a day.

[Results]

Table 4 summarizes the results.

On the next day of the completion of the test period (two weeks), the axillary part, to which the invention product had been applied, was compared with another one, to which the control product had been applied. In Table 4, "remarkably effective" means a case wherein the score of the test part is lower by three grades than that of the control part; "effective" means a case wherein the former score is lower than the latter one by two grades; "somewhat effective" means a case wherein the former score is lower than the latter one by one grade; and "ineffective" means other cases.

Table 4

| | |
|---|---|
| remarkably effective | 5 (50%) |
| effective | 2 (20%) |
| somewhat effective | 2 (20%) |
| ineffective | 1 (10%) |
| in total | 10 (100%) |

Example 3

A deodorant spray of each composition as specified in Table 5 was produced in a conventional manner. Then these sprays were subjected to the same test as the one described in Example 1 and the results thus obtained were evaluated according to the same criteria as those specified in Example 1. Table 6 summarizes the results.

Table 5: (Composition of deodorant spray: % by weight)

| Ingredient | Invention product 3 | Comparative product 3 |
|---|---|---|
| β-glycyrrhetic acid | 1.0 | - |
| ethanol | 40.0 | 40.0 |
| fluorocarbon 11 | 53.1 | 54.1 |
| fluorocarbon 12 | 5.9 | 5.9 |
| in total | 100.0 | 100.0 |

Table 6: (No. of subjects)

| Axillary odor | Invention product 3 | | Comparative product 3 | |
|---|---|---|---|---|
| | before treatment | after treatment | before treatment | after treatment |
| ++ | 2 | 0 | 2 | 2 |
| + | 3 | 0 | 3 | 3 |
| ± | 0 | 0 | 0 | 0 |
| - | 0 | 5 | 0 | 0 |

Example 4

A deodorant spray (the invention product 4) of the stock solution composition and the filling composition each specified in Table 7 was produced by the following method.

Table 7: (% by weight)

| | | |
|---|---|---|
| Stock solution composition | glycyrrhetic acid | 1.5 |
| | aluminum hydroxychloride | 10.0 |
| | isopropyl myristate | 3.0 |
| | perfume | 0.2 |
| | ethanol | 85.3 |
| | in total | 100.0 |
| Filling composition | stock solution | 36.0 |
| | LPG | 64.0 |
| | in total | 100.0 |

[Production Method]

The stock solution was prepared by dissolving aluminum hydroxychloride in ethanol and adding other ingredients to the resulting solution followed by filtering. A definite amount of the stock solution was fed into a can followed by providing a valve. Next, a definite amount of LPG was fed to the can to thereby give a deodorant spray.

Example 5

A deodorant stick (the invention product 5) of the composition as specified in Table 8 was produced by the following method.

Table 8: (composition of deodorant stick: % by weight)

| | |
|---|---|
| glycyrrhetic acid | 0.5 |
| zinc white | 12.0 |
| solid paraffin*1 | 12.0 |
| beeswax | 23.0 |
| vaseline*2 | 23.0 |
| liquid paraffin*3 | 29.3 |
| perfume | 0.1 |
| BHT | 0.1 |
| in total | 100.0 |

*1: Trademark: Solid Paraffin 125F.

*2: Trademark: White Protopet IS.

*3: Trademark: Silicol P-55.

[Production Method]

The above ingredients other than zinc white were mixed together and molten by heating. Then zinc white was added thereto and the obtained mixture was homogeneously dispersed in a homomixer. After pouring into a mold, the dispersion was quenched to thereby give a deodorant stick.

**Claims**

1. A deodorant for axillary odor comprising glycyrrhetic acid.

2. A deodorant for axillary odor as claimed in Claim 1, wherein the content of glycyrrhetic acid ranges from 0.01 to 5% by weight.

3. A deodorant for axillary odor as claimed in Claim 1, which contains one or more ingredients for preventing body odor selected from a group consisting of antiperspirants, bactericides and mask agents.

4. A deodorant for axillary odor as claimed in Claim 3, wherein said antiperspirant is selected from a group consisting of aluminum hydroxychloride, aluminum chloride, aluminum sulfate, basic aluminum

bromide, aluminum phenolsulfonate, tannic acid, aluminum naphthalenesulfonate and basic aluminum iodide.

**5.** A deodorant for axillary odor as claimed din Claim 3, wherein said bactericide is selected from a group consisting of 2,4,4'-trichloro-2'-hydroxydiphenyl ether, 3,4,4-trichlorocarbanilide, benzalkonium chloride, benzethonium chloride, alkyltrimethylammonium chlorides, resorcin, phenol, sorbic acid, salicylic acid and hexachlorophene.

**6.** A deodorant for axillary odor as claimed in Claim 3, wherein said masking agent is selected from a group consisting of musk, skatole, lemon oil, lavender oil, absolute, jasmine, vanillin, benzoin, benzyl acetate and menthol.

European Patent Office

# EUROPEAN SEARCH REPORT

Application Number

**EP 90 12 4243**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-2 523 866 (R. GREVE)<br>* Claims; page 4, paragraphs 1-4 *<br>- - - | 1-6 | A 61 K 7/32 |
| Y | FR-A-2 509 173 (M. LAVOILLOTTE)<br>* Claims; page 2, line 12 - page 5, line 38 *<br>- - - - - | 1-6 | |
| | | | **TECHNICAL FIELDS SEARCHED (Int. Cl.5)** |
| | | | A 61 K |

The present search report has been drawn up for all claims

| Place of search | Date of completion of search | Examiner |
|---|---|---|
| The Hague | 25 February 91 | WILLEKENS G.E.J. |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document